# EUROPEAN PATENT SPECIFICATION

(11) **EP 3 815 710 B1**
(45) Date of publication and mention of the grant of the patent: **20.12.2023**
(21) Application number: 20203130.8
(22) Date of filing: 21.10.2020
(51) Int. Cl.: A61K 38/48, A61P 29/00

(54) **SERRAPEPTASE FOR USE IN THE TREATMENT OF DEEP ENDOMETRIOSIS**
SERRAPEPTASE ZUR BEHANDLUNG VON TIEFER ENDOMETRIOSE
SERRAPEPTASE POUR LE TRAITEMENT DE L'ENDOMETRIOSE PROFONDE

(30) Priority: 28.10.2019 IT 201900019876
(43) Date of publication of application: 05.05.2021
(73) Proprietor: Napoletano, Ilaria, 04100 Latina (LT) (IT)
(72) Inventor: Napoletano, Ilaria, 04100 Latina (LT) (IT)
(74) Representative: Perani & Partners S.p.A.

(56) References cited:
- EP-A1- 3 222 283
- EP-A2- 0 136 781
- US-A1- 2015 359 860
- ANONYMOUS: "Neprinol Sever Inflammation Defense", INTERNET CITATION, 17 March 2008 (2008-03-17), XP002473110, Retrieved from the Internet: URL:http://www.crohns-disease-probiotics.c om/neprinol.html [retrieved on 2008-03-17]
- Lova: "Serrapeptase,nattokinase, wobenzymreview forendometriosis pain", Endometriosis Healing , 27 November 2014 (2014-11-27), XP002802310, Retrieved from the Internet: URL:https://endometriosishealing.wordpress .com/2014/11/27/serrapeptase-nattokinase-w obenzym-review-for-endometriosis-pain/ [retrieved on 2021-03-09]

## Description

### FIELD OF THE INVENTION

The present invention relates to the use of serrapeptase, in particular administered orally together with shiitake and even more preferably together with shiitake and reishi in the treatment of deep endometriosis.

### BACKGROUND ART

Endometriosis is still a little-known disease which affects women of childbearing age: it is estimated that 3 million women are affected in Italy alone, even 1 in 2 in the 29-39 age group. One of the main problems related to endometriosis is the difficulty of diagnosis: all too often, the correct diagnosis is reached after countless cycles of therapy aimed at the treatment of other types of diseases.

In fact, the increasing pains associated with the menstrual cycle are considered physiological and therapeutic investigations are postponed.

Endometriosis is often the cause of infertility, pelvic pain, dyspareunia, and is characterized by the ectopic presence outside the uterine cavity of endometrial tissue which can lead to adhesions, cystic formations, abdominal bleeding.

If endometriosis is an etiologic and therapeutic/surgical problem, deep or infiltrating endometriosis is an even greater problem.

Deep or infiltrating endometriosis is that type of endometriosis whereby the pathological tissue penetrates more than 5 mm into the affected anatomical structures. The most frequent location of this type of disease is the intestines and the most frequently affected tracts are the recto-sigma junction and the rectum, the part of the intestine in direct contact with vagina and from which it is separated by the recto-vaginal septum.

There are various kinds of symptoms of deep endometriosis and include, in addition to abdominal pain and dyspareunia (felt deeply both during and after sexual intercourse), changes in the alvo such as diarrhoea, intestinal bleeding, which sometimes appear or worsen during menstruation.

The diagnosis of deep endometriosis, rather than with a gynaecological examination, which only detects typical alterations of the fornix and/or recto-vaginal septum in some cases, finds valid aid both with transvaginal pelvic ultrasound and through magnetic resonance imaging (MRI) of the pelvis. Sometimes, dual contrast opaque enema, conventional and virtual colonoscopy may also be helpful.

The presence of occult blood in the faeces can also testify micro-bleeding in the most severe forms of deep endometriosis, whereby the pathological tissue infiltrates the intestinal mucosa and the typical CA125 blood marker can be used to diagnose this form of endometriosis.

In these cases it is difficult for the doctor to choose the best therapy if the general conditions of the patient, the impact of the quality of life, the history and characteristics of the endometriosis disease, as well as, of course, the extension thereof, are not known.

Where surgery is required, the procedure is laparoscopic and very complex, so that it should only be performed in highly specialized centres. In fact, deep endometriosis often requires bowel resection and is burdened by a considerable number of complications, including bladder and rectal dysfunctions resulting from denervation. The simultaneous presence of endometriosis outbreaks in other sites, sometimes with cyst formation, increases the difficulties of these interventions, which sometimes require the passage to the laparotomic path and the assistance of a general surgeon.

Drug therapy is often able to reduce painful symptoms and the size of lesions. Sometimes it is administered to the patient before surgery to reduce active lesions and make it more conservative; however, if it is administered after surgery and lasts for long periods of time, it can reduce the number of relapses.

The most commonly used drugs in deep endometriosis are:
- Combined oral contraceptives including the most recent extended-regimen contraceptives (SEASONIQUE^{®}) taken in 91-day cycles;
- GnRH agonists (DECAPEPTYL^{®}, capable of causing a temporary menopause), which is very effective but aggravated by side effects caused by hypoestrogenism;
- Low-dose progestins administered continuously (Norethisterone acetate, 5 mg, 7 days, or Dienogest for the antiproliferative action on both the ectopic and endouterine endometrium,
- Danazole has also been used in direct intravaginal administration, but should be avoided for long periods of time for the androgenic side effects (F. Di Prospero "Endometriosi profonda infiltrante"26.12.2018 (https://fertihelp.it/endometriosi/endometriosi-profonda-infiltrante/)

Therefore, long periods of drug therapy, as highlighted above, can also lead to a series of side effects.

The applicant of the present patent has already filed patent 102016000029331which discloses the use of shiitake extract, even better if associated with reishi, for the treatment and prevention of endometriosis.

Serrapeptase (also known as Serratiopeptidase or Serratia E-15 protease or Serralysin) is a proteolytic enzyme (a protease) produced and obtained from some species of Serratia, an enterobacterium. This microorganism was originally isolated in the late 1960s from the Bombyx mori silkworm. Serrapeptase is present in the intestine of the silkworm and allows the moth to emerge by dissolving its cocoon. Serrapeptase is manufactured starting from the purification of Serratia E-15 bacterial cultures.

The enzyme has an anti-inflammatory, anti-oedema, proteolytic and fibrinolytic action. Most of the effects are related to the anti-radiquinine activity.

As is known, bradykinin is a peptide hormone formed by 9 amino acid residues, which at the level of inflammatory outbreaks causes an intense vasodilation action and an increase in capillary permeability. The peptide is also associated with leukocyte migration to the site of inflammation and intense local sensations of pain.

Thanks to its proteolytic and fibrinolytic activity, serrapeptase facilitates the demolition and dissolution of fibrinous and protein exudates associated with the inflammatory process. It also simplifies the resorption of any haematomas, and by improving local circulation, facilitates the penetration of other drugs (e.g., antibiotics),[1][2][3][4][5][6] helping to facilitate the elimination of collimated material and improving regenerative and tissue repair processes.[7] The enzyme also shows fluidizing action of mucous and purulent secretions. [8]

Serrapeptase is proposed in oedema processes of any nature, particularly post-traumatic and post-operative. [9] The compound has been tested in the treatment of phlebopathies, varicose ulcers, breast engorgement. [10] The drug is also proposed in the symptomatic treatment of inflammatory reactions associated with upper and lower airway infections.

In a 1984 double-blind, multi-centre study of a total of 174 patients undergoing Caldwell-Luc antrostomy for chronic empyema, the extent of oedema in serrapeptase-treated patients was significantly lower than in the control group. [11] US2015359860 discloses a method for dissolving proteins deposited on muscles comprising administering an effective amount of an agent selected from fibrinolytic, proteolytic, photolytic, and magnelithic agents, such as serrapeptase.

The document: ANONYMOUS: "Neprinol Sever Inflammation Defense",INTERNET CITATION, 17 March 2008 describes the benefits of Neprinol^{®}, which is a trademark of a product which groups a series of anti-inflammatory enzymes including serrapeptase which act in synergy to reduce ulcerative colitis, Crohn's disease and also a series of inflammatory diseases including endometriosis. However, no experimental data is provided for this type of disease.

### SUMMARY OF THE INVENTION

The applicant has now found that serrapeptase can be advantageously used in the treatment of deep endometriosis.

Therefore, the object of the present invention relates to the use of serrapeptase in the treatment of this disease.

In particular, serrapeptase for the use according to the present invention according to the present invention is administered orally in formulations containing it as an active ingredient in combination with suitable excipients and/or diluents for oral formulation.

### DETAILED DESCRIPTION OF THE INVENTION

For the purposes of the present invention, the definition "containing" or "comprising" does not exclude the possibility of additional components being contained in addition to those listed after said definition, while the definition "consisting of" excludes such an eventuality.

Preferably, serrapeptase is administered as conventional oral formulations such as, for example, tablets, capsules and water-dispersible powders.

Serrapeptase for the use according to the present invention is preferably administered orally at dosages ranging from 60,000 to 250,000 IU once to twice a day, more preferably 120,000 IU once a day.

Preferably, the oral administration of serrapeptase for the use according to the present invention also contemplates the oral administration of shiitake extract and possibly also of reishi extract. Even more preferably, the serrapeptase for use according to the present invention is administered together with both of the above extracts.

In this case, said shiitake and reishi extracts are themselves contained in the same oral formulation as that containing serrapeptase or in one or two oral formulations different from that of serrapeptase, preferably they are contained in only one oral formulation distinct from those of serrapeptase.

These oral formulations are preferably food supplements. For the purposes of the present invention, the definition of food supplement means those formulations which fall under Directive 2002/46/EC and subsequent amendments. In this legislation, food supplements are precisely defined as: 'foodstuffs intended to supplement the common diet and constituting a concentrated source of nutrients, such as vitamins and minerals, or of other substances having a nutritional or physiological effect, in particular, but not exclusively, amino acids, essential fatty acids, fibres and extracts of plant origin, both mono- and multi-compound, in predominant forms'.

Preferably, the shiitake extract for the use according to the present invention is administered at daily dosages between 100 and 1000 mg, more preferably between 500mg and 800 mg.

Preferably, also the reishi extract, when present, is administered at daily dosages ranging from 100 to 1000 mg, more preferably from 500 to 800mg.

The results obtained with the use of serrapeptase according to the present invention are set forth below in the following example.

### EXAMPLE 1

A 25-year-old patient with deep endometriosis was treated with Dienogest for 3 months, then this treatment was discontinued as the patient reported problems of excessive heat, fatigue, hair loss, weight gain, increased water retention, headache, dry mouth, mood swings, while changes in blood clotting factors had also been found. Subsequently, the patient was treated with a combination of shiitake and reishi extracts for 36 months, administered at daily doses of 700mg each in the form of a single food supplement in capsules containing 350 mg each of the aforementioned active ingredients 2 times a day, from whose therapy she benefited considerably. In fact, after this therapy, the patient underwent oocyte pick-up for FIVET surgery, which was successful even after the first attempt and she became pregnant. After the birth of the child in July 2016, however, this therapy was not sufficient, since the patient reported very severe post-partum pain.

For this reason, shiitake and reishi therapy was supplemented with serrapeptase at a rate of 120,000 units administered once daily as a single capsule.

After 3 months of this therapy an MRI analysis of the pelvic cavity was performed.

This examination was performed on 3T magnet following intravenous injection of spasmolytic drug (BUSCOPAN), using T1 and T2 weighted multiplanar sequences, in diffusion with and without adipose tissue signal saturation under baseline conditions only. This check was carried out on the patient suffering from endometriosis, currently asymptomatic. The MRI shows:
- a uterus of normal size with normal flexion, with substantially homogeneous myometric signal strength;
- endometrial thickness within normal limits;
- the fibro-endometric plate, described in the previous MRI, remains substantially unchanged in size and morphology in the retro-cervical area at the level of the uterine torus, which obliterates the recto-uterine pouch and infiltrates the front side of the sigma-rect hemi-circumferential plate junction extending longitudinally for about 3 cm, maximum parietal thickness of about 9 mm.
- In these sites, there is a current reduction in contextual active bleeding outbreaks.
- The active outbreak of endometriosis with previously visible linear morphology at the uterine serosa on the right anterolateral side is not clearly recognizable.
- Ovaries of sizes within normal limits free from endometriosis cysts.
- Modest pericardium effusion in the recto-uterine pouch and in the left parauterine site.

### Subsequent MRI was performed 6 months later then 9 months after initiation of serrapeptase therapy

This examination was also performed on 3T magnet following intravenous injection of spasmolytic drug (BUSCOPAN), using T1 and T2 weighted multiplanar sequences, in diffusion with and without adipose tissue signal saturation under baseline conditions only. The comparison was made with the previous magnetic resonance imaging examination.

The results were compared with the previous MRI conducted 6 months before and summarized above. The patient still does not report any symptoms of endometriosis. This analysis highlights:
- a uterus of normal size with antiverse flexion, with substantially homogeneous myometric signal intensity;
- slight thickening of the junction line, in the absence of foci of hyperintensity in the T1 sequences weighed as for secreting adenomyosis;
- endometrial thickness within normal limits.
- the fibro-endometric plate, described in the previous MRI, remains substantially unchanged in size and morphology in the retro-cervical area at the level of the uterine torus, which obliterates the recto-uterine pouch and infiltrates the front side of the sigma-rect hemi-circumferential plate junction extended longitudinally for about 3 cm, maximum parietal thickness of about 9 mm,
- at this point, however, no confirmed, active endometriosis foci are visible.
- A small outbreak of active endometriosis is detected again, probably located in the left perivesical adipose tissue, in the perivesical tract of the same.
- Ovaries of sizes within normal limits free from endometriosis cysts.
- Small spillage flap arranged on the right of the unchanged rectum uteri pouch.
- The focal thickening of the anterior bladder wall of a probable fibrous nature remains unchanged.

### Conclusions

It is therefore noted that serrapeptase is very effective both in reducing the typical symptoms of endometriosis,and in particular pain. These results are also confirmed by the analytical results, which show a substantial reduction in active outbreaks, while both the pre-existing fibrous thickening of the bladder wall and the size of the fibro-endometric plate and the pre-existing effusion remain unchanged.

### Bibliografia

H. Aratani, S. Kono; Y. Yamanaka, [Fundamental studies on the distribution of sulfobenzylpenicillin. Effect of serratiopeptidase (authorstransl)]., in Jpn J Antibiot, vol. 27, no.3, Jun 1974, pp.271-8,PMID 4436946.
1. H. Okumura, R. Watanabe; Y. Kotoura; Y. Nakane; O. Tangiku, [Effects of a proteolytic-enzyme preparation used concomitantly with an antibiotic in osteoarticular infections (authors' transl)]., in Jpn J Antibiot, vol. 30, no.3, Mar 1977, pp.223-7,PMID 853579.
2. H. Aratani, H. Tateishi; S. Negita, [Studies on in vivo activity of antibiotics in experimental pneumonia in rats. I. Combination of cyclacillin and serratiopeptidase (authorstransl)]., in Jpn J Antibiot, vol. 32, No.8, Aug. 1979, pp.806-1 1,PMID 490898.
3. H. Aratani, H. Tateishi; S. Negita, [Studies on the distributions of antibiotics in the oral tissues: Experimental staphylococcal infection in rats, and effect of serratiopeptidase on the distributions of antibiotics (authors' transl)]., in Jpn J Antibiot, vol. 33, No.5, May 1980, pp.623-35,PMID 7001087.
4. A. Koyama, J. Mori; H. Tokuda; M. Waku; H. Anno; T. Katayama; K. Murakami; H. Komatsu; M. Hirata; T. Arai,[Augmentation by serrapeptase of tissue permeation by cefotiam]., in Jpn J Antibiot, vol. 39, No.3, Mar 1986, pp.761-71,PMID 3525882.
5. M. Mecikoglu, B. Saygi; Y. Yildirim; E. Karadag-Saygi; SS. Ramadan; T. Esemenli,,, in J Bone Joint Surg Am, vol. 88, no.6, June 2006, pp.1208-14,:,.The effect of proteolytic enzyme serratiopeptidase in the treatment of experimental implant-related infection.DOI.10.2106/JBJS.E.00007PMID 16757752
6. Sannino, P. Gigola; M. Puttini; F. Pera; C. Passariello,Combination therapy including serratiopeptidase improves outcomes of mechanical-antibiotic treatment of periimplantitis., inInt J Immunopathol Pharmacol, vol. 26, no.3, pp.825-3 EPMID 24067485.
7. Kasé, H. Seo; Y. Oyama; M. Sakata; K. Tomoda; K. Takahama; T. Hitoshi; Y. Okano; T. Miyata,A new method for evaluating mucolytic expectorant activity and its application. II. Application to two proteolytic enzymes, serratiopeptidase and seaprose., inArzneimittelforschung, vol. 32, No.4, 1982, pp.374-8,PMID 7049188.
8. PM. Esch, H. Gerngross; A. Fabian,[Reduction of postoperative swelling. Objective measurement of swelling of the upper ankle joint in treatment with serrapeptase-- a prospective study]., inFortschr Med, vol. 107, No.4, Feb 1989, pp.67-8, 71-2,PMID 2647603.
9. WH. Kee, SL. Tan; V. Lee; YM. Salmon, The treatment of breast engorgement with Serrapeptase (Danzen): a randomised double-blind controlled trial., inSingapore Med J, vol. 30, no.1, Feb 1989, pp.48-54,PMID 2688125.
10. M. Tachibana, O. Mizukoshi; Y. Harada; K. Kawamoto; Y. Nakai,A multi-centre, double-blind study of serrapeptase versus placebo in post-antrotomy buccal swelling., inPharmatherapeutica, vol. 3, no.8, 1984, pp.526-30,PMID 6366808.

## Claims

1. Serrapeptase for use in the treatment of deep endometriosis.

2. Serrapeptase for the use according to claim 1, wherein said serrapeptase is orally administered in formulations containing it as an active ingredient in combination with suitable excipients and/or diluents.

3. Serrapeptase for the use according to claim 2, wherein said serrapeptase is administered orally at dosages ranging from 60,000 to 250,000 IU, from one to two times a day.

4. Serrapeptase for the use according to claim 3, wherein said serrapeptase is administered at dosages of 120,000 IU once a day.

5. Serrapeptase for the use according to any one of claims 2-4, wherein said serrapeptase is administered in association with an oral administration of shiitake extract, possibly together with reishi extract.

6. Serrapeptase for the use according to claim 5, wherein said oral administration of shiitake extract is associated with an oral administration of said reishi extract.

7. Serrapeptase for the use according to claim 6, wherein said shiitake and reishi extracts are contained on their turn in the same oral formulation as the one containing serrapeptase or in one or two oral formulations different from the one comprising serrapeptase, preferably said shiitake and reishi are contained in a single oral formulation distinct from the one containing serrapeptase.

8. Serrapeptase for use according to any one of claims 5-7, wherein said shiitake extract is administered at daily doses of between 100 and 1000 mg, preferably 500 mg.

9. Serrapeptase for the use according to any one of claims 5-7, wherein said reishi extract is administered at daily dosages of between 100 and 1000 mg, preferably between 500 mg and 800 mg.

10. Serrapeptase for the use according to any one of claims 7-9, wherein said oral formulation (s) are food supplements.

## Patentansprüche

1. Serrapeptase zur Verwendung bei der Behandlung der tiefen Endometriose.

2. Serrapeptase zur Verwendung nach Anspruch 1, wobei die Serrapeptase in Formulierungen, die sie als Wirkstoff in Kombination mit geeigneten Hilfsstoffen und/oder Verdünnungsmitteln enthalten, oral verabreicht wird.

3. Serrapeptase zur Verwendung nach Anspruch 2, wobei die Serrapeptase in Dosierungen im Bereich von 60.000 bis 250.000 IE ein- bis zweimal täglich oral verabreicht wird.

4. Serrapeptase zur Verwendung nach Anspruch 3, wobei die Serrapeptase in Dosierungen von 120.000 IE einmal täglich verabreicht wird.

5. Serrapeptase zur Verwendung nach einem der Ansprüche 2 bis 4, wobei die Serrapeptase in Verbindung mit einer oralen Verabreichung von Shiitake-Extrakt, gegebenenfalls zusammen mit Reishi-Extrakt, verabreicht wird.

6. Serrapeptase zur Verwendung nach Anspruch 5, wobei die orale Verabreichung von Shiitake-Extrakt mit einer oralen Verabreichung des Reishi-Extrakts verbunden ist.

7. Serrapeptase zur Verwendung nach Anspruch 6, wobei die Shiitake- und Reishi-Extrakte ihrerseits in derselben oralen Formulierung enthalten sind wie die, die Serrapeptase enthält, oder in einer oder zwei oralen Formulierungen, die sich von der unterscheiden, die Serrapeptase enthält, wobei vorzugsweise die Shiitake- und Reishi-Extrakte in einer einzigen oralen Formulierung enthalten sind, die sich von der unterscheidet, die Serrapeptase enthält.

8. Serrapeptase zur Verwendung nach einem der Ansprüche 5 bis 7, wobei das Shiitake-Extrakt in Tagesdosen zwischen 100 und 1000 mg, vorzugsweise 500 mg, verabreicht wird.

9. Serrapeptase zur Verwendung nach einem der Ansprüche 5 bis 7, wobei das Reishi-Extrakt in täglichen Dosierungen zwischen 100 und 1000 mg, vorzugsweise zwischen 500 mg und 800 mg, verabreicht wird.

10. Serrapeptase zur Verwendung nach einem der Ansprüche 7 bis 9, wobei die orale(n) Formulierung(en) Nahrungsergänzungsmittel ist (sind).

## Revendications

1. Serrapeptase pour l'utilisation dans le traitement de l'endométriose profonde.

2. Serrapeptase pour l'utilisation selon la revendication 1, dans laquelle ladite serrapeptase est administrée par voie orale dans des formulations la contenant en tant qu'ingrédient actif en combinaison avec des excipients et/ou des diluants appropriés.

3. Serrapeptase pour l'utilisation selon la revendication 2, dans laquelle ladite serrapeptase est administrée par voie orale à des dosages allant de 60 000 à 250 000 UI, d'une à deux fois par jour.

4. Serrapeptase pour l'utilisation selon la revendication 3, dans laquelle ladite serrapeptase est administrée à des dosages de 120 000 UI une fois par jour.

5. Serrapeptase pour l'utilisation selon l'une quelconque des revendications 2 à 4, dans laquelle ladite serrapeptase est administrée en association avec une administration orale d'extrait de shiitake, éventuellement avec de l'extrait de reishi.

6. Serrapeptase pour l'utilisation selon la revendication 5, dans laquelle ladite administration orale d'extrait de shiitake est associée à une administration orale dudit extrait de reishi.

7. Serrapeptase pour l'utilisation selon la revendication 6, dans laquelle lesdits extraits de shiitake et de reishi sont contenus à leur tour dans la même formulation orale que celle contenant la serrapeptase ou dans une ou deux formulations orales différentes de celle comprenant la serrapeptase, de préférence lesdits shiitake et reishi sont contenus dans une formulation orale unique distincte de celle contenant la serrapeptase.

8. Serrapeptase pour l'utilisation selon l'une quelconque des revendications 5 à 7, dans laquelle ledit extrait de shiitake est administré à des doses quotidiennes comprises entre 100 et 1000 mg, de préférence 500 mg.

9. Serrapeptase pour l'utilisation selon l'une quelconque des revendications 5 à 7, dans laquelle ledit extrait de reishi est administré à des dosages quotidiennes comprises entre 100 et 1000 mg, de préférence entre 500 mg et 800 mg.

10. Serrapeptase pour l'utilisation selon l'une quelconque des revendications 7 à 9, dans laquelle la (les) formulation(s) orale(s) est (sont) compléments alimentaires.
